# EUROPEAN PATENT SPECIFICATION

(11) **EP 0 868 921 B1**
(45) Date of publication and mention of the grant of the patent: **30.06.2004**
(21) Application number: 98302583.4
(22) Date of filing: 02.04.1998
(51) Int. Cl.: A61K 49/00, A61K 33/00, A61P 35/00

(54) **Medical gas mixtures**
Medizinische Gasmischungen
Mélanges de gaz médicaux

(30) Priority: 04.04.1997 GB 9706817
(43) Date of publication of application: 07.10.1998
(73) Proprietor: The BOC Group plc, Windlesham Surrey GU20 6HJ (GB)
(72) Inventor: Garrett, Michael Ernest, Woking, Surrey GU22 7XR (GB)
(74) Representative: Bousfield, Roger James

(56) References cited:
- EP-A- 0 551 898
- EP-A- 0 727 219
- ROBINSON, S. P. ET AL.: "Noninvasive Monitoring of Carbogen-Induced Changes In Tumor Blood Flow and Oxygenation by Functional Magnetic Resonance Imaging " INTERNATIONAL JOURNAL OF RADIATION ONCOLOGY, BIOLOGY, PHYSICS, vol. 33, no. 4, 1995, pages 855-859, XP002097897
- ROBINSON S. P. ET AL.: "The Response to Carbogen Breathing in Experimental Tumour Models Monitored by Gradient-Recalled echo Magnetic Resonance Imaging" BRITISH JOURNAL OF CANCER, vol. 75, no. 7, April 1997, pages 1000-1006, XP002097898
- ORSINI A. J. ET AL.: "Helium-Oxygen Gas Mixtures Decrease Resistance and Improve Ventilation in Neonates with Respiratory Distress Syndrome" PEDRIATIC RESEARCH , vol. 39, no. 4 (part 2), 1996, page 344 A XP002097899

## Description

This invention relates to medical gas mixtures and, more particularly, to such mixtures for use with patients undergoing imaging, for example magnetic resonance imaging (MRI).

In MRI detection of cancer, the presence of oxygenated blood within the tumour can commonly improve the MRI imaging.

This can be achieved in particular by there being an enhanced level of oxygen in the gas/atmosphere being inhaled by the patient. In addition it has been found that the presence of carbon dioxide in the inhaled gas acts as a vasodilator such that, in the area of the tumour in particular, dilated blood vessels in and around the tumour allow for an increased level of oxygen in those blood vessels. Typically a five percent addition of carbon dioxide (CO₂) is made to the gas although somewhat higher or lower concentration may be used to good effect in different circumstances.

The carbon dioxide has in practice been administered in air or oxygen enriched air as the inhaled gas but more commonly it is administered in oxygen (O₂) in particular by inhalation of a 5% CO₂/95% O₂ mixture. Such a mixture is commercially available from the BOC Gases business of the Applicant under the trade name "Carbogen".

However, a problem with the administration of Carbogen mixtures to patients is that pronounced physiological effects at a 5% CO₂ concentration, including inability to breath normally, high ventilation rates, panic and production of heat which may lead to the patient being unable or unwilling to continue with the imaging or treatment. This is particularly the case for patients with obstructive pulmonary disease.

It has been recently proposed to reduce the carbon dioxide concentration somewhat. For example down to 4% or 3% or lower in order to minimise such detrimental physiological effects and it is thought that this may be possible without substantial loss of the benefits in the imaging process of inhaling Carbogen gas mixtures.

Nevertheless, the need to allow a patient to inhale such gas mixtures with a minimum of discomfort and lack of the above physiological effects remains paramount if the best possible imaging to be consistently obtained.

EP-A-0727219 in the name of the present Applicants discloses a medical gas mixture with particular reference to the treatment of asthma comprising 20% to 40% oxygen, 2% to 10% carbon dioxide and the balance being hydrogen.

EP-A-0551898 discloses a method of radiological imaging of patients, comprising providing a stable gas mixture of 20 to 75.5 mole % xenon, 19.5 to 75 mole % oxygen, 5 to 60.5 mole % helium and up to 10 mole % carbon dioxide.

A paper entitled "Noninvasive Monitoring of Carbogen-Indued Changes in Tumor Blood Flow and Oxygenation by Functional Magnetic Resonance Imaging" International Journal of Radiation Oncology, Biology, Physics, vol. 33, no. 4, 1995, pages 855-859 by S P Robinson et al discloses that the response of tumours to radiotherapy can be enhanced by breathing a "Carbogen" gas mixture comprising 95% oxygen and 5% carbon dioxide.

A further paper by SP Robinson et al entitled "The Response to Carbogen Breathing in Experimental Tumour Models Monitored by Gradient-Recalled echo Magnetic Resonance Imaging" British Journal of Cancer, vol. 75, no. 7 April 1997, pages 1000-1006 describes the response of different tumours by magnetic resonance imaging (MRI) in patients breathing a "Carbogen" gas mixture.

A paper by A J Orsini et all entitled "Helium-Oxygen Gas Mixtures Decrease Resistance and Improve Ventilation in Neonates with Respiratory Distress Syndrome" Pedriatic Research vol. 39, no. 4 (part 2), 1996, page 334A discloses that a helium-oxygen gas mixture lowers resistance and improves ventilation at lower transpulmonic pressures.

The present invention is concerned with the provision of new medical gas mixtures for use in particular in MRI imaging.

In accordance with the invention there is provided a medical gas mixture which comprises from 20 to 70% oxygen, from 1 to 8% carbon dioxide, the balance being helium.

All percentages quoted in this specification and claims are, unless otherwise indicated, by volume.

The presence of helium, a relatively light gas, surprisingly allows the mixture as a whole to be more readily and comfortably inhaled than mixtures previously used.

The effect of helium is to greatly reduce the "drag" in a patient's lungs caused by the high breathing rate triggered by the carbon dioxide constituent and at the same time to improve the perfusion of the inhaled gas constituents - oxygen and carbon dioxide - in to the depths of the lungs.

The presence of helium therefore affords the possibility of using somewhat lower carbon dioxide concentration than the typical 5% content in Carbogen mixtures. The carbon dioxide is preferably from 1 to 5%, more preferably from 1 to 4% and ideally less than 3%.

The oxygen content may be varied over the broad range stipulated above but is preferably from 20 to 40% and more preferably from 25 to 35%, for example 30 or 31%. It has been found in general that little extra benefit in MRI imaging in particular occurs at oxygen concentrations above about 31% as no extra arterial oxygen is introduced with higher concentrations.

The lower concentration of oxygen in the mixtures of the invention as compared with use of standard Carbogen mixtures may also be beneficial in reducing oxygen saturation in the plasma of the blood, thereby improving the oxyhaemoglobin image in MRI in particular.

An overall preferred gas mixture contains from 25 to 35% oxygen, 1 to 3% carbon dioxide and the balance being helium, for example 30% oxygen, 2% carbon dioxide and 68% helium.

The basic properties exhibited by the gas mixture of the invention, namely good blood oxygenation and vasodilation in the patient inhaling the mixture together is easier breathing characteristics for the patient, allows the mixture to be used to good effect not only for enhanced imaging procedures and results but also in the associated treatment of the tumours in question themselves.

In this letter respect, it has been surprisingly found that the gas mixture of the invention can greatly assist in the chemotherapy and radiotherapy treatment of tumours. In particular, the improved imaging effects afforded by the mixture based in the presence of enhanced levels of oxygen can show more clearly those tumours and parts of tumours in which blood is still flowing, i.e. have not been necrotic, and that are therefore susceptible to chemotherapy and/or radiology. Furthermore, the vasodilation properties of the gas mixtures of the invention allow, during inhalation by the patient, for the chemotherapy and/or radiotherapy drags to be more widely available within the tumour in particular and to be retained or trapped in the tumour once the inhaler has ceased and the blood vessels constrict.

The invention can therefore be regarded as the use of the gas mixture of the invention in tumour imaging techniques and in the associated treatments of the tumours.

Experiments have shown that the use of the relatively low levels of carbon dioxide, particularly less than 3%, surprisingly do not decrease the vasodilation effects of this constituent, nor adversely affect the overall effectiveness of the imaging or patients inhaling the gas mixture, to an extent which would be expected for such low levels of carbon dioxide.

In addition the experiments confirm the fact that the gas mixture of the invention is more readily and comfortably inhaled than an equivalent helium-free mixture.

## Claims

1. The use of a medical gas mixture in the preparation of a medicament for the imaging and/or treatment of tumours in patients inhaling the mixture, the mixture comprising for 20 to 70% oxygen, from 1 - 8% carbon dioxide, the balance being helium.

2. The use of a medical gas mixture according to Claim 1 in that the oxygen content is from 20 to 40%.

3. The use of a medical gas mixture according Claim 1 or Claim 2 in which the oxygen content is from 25 to 35%.

4. The use of a medical gas mixture according to any preceding claim in which the carbon dioxide content is from 1 to 5%.

5. The use of a medical gas mixture according to any preceding claim in which the carbon dioxide content is from 1 to 4%.

6. The use of a medical gas mixture according to any preceding claim in which the carbon dioxide is less than 3%.

7. The use of a medical gas mixture according to Claim 1 containing 25 to 35% oxygen, 1 to 3% carbon dioxide and the balance being helium.

8. The use of a medical gas mixture according to Claim 7 containing 30% oxygen, 2% carbon dioxide and 68% helium.

## Patentansprüche

1. Verwendung eines medizinischen Gasgemischs bei der Zubereitung eines Medikaments für die Abbildung und/oder Behandlung von Tumoren in Patienten, welche das Gemisch einatmen, wobei das Gemisch 20 bis 70% Sauerstoff, 1 bis 8 % Kohlendioxid und im übrigen Helium aufweist.

2. Verwendung eines medizinischen Gasgemischs nach Anspruchs 1, wobei der Sauerstoffgehalt 20 bis 40% beträgt.

3. Verwendung eines medizinischen Gasgemischs nach Anspruch 1 oder Anspruch 2, wobei der Sauerstoffgehalt 25 bis 35% beträgt.

4. Verwendung eines medizinischen Gasgemischs nach einem der vorhergehenden Ansprüche, wobei der Kohlendioxidgehalt 1 bis 5% beträgt.

5. Verwendung eines medizinischen Gasgemischs nach einem der vorhergehenden Ansprüche, wobei der Kohlendioxidgehalt 1 bis 4% beträgt.

6. Verwendung eines medizinischen Gasgemischs nach einem der vorhergehenden Ansprüche, wobei der Kohlendioxidgehalt weniger als 3% beträgt.

7. Verwendung eines medizinischen Gasgemischs nach Anspruch 1 mit 25 bis 35% Sauerstoff, 1 bis 3% Kohlendioxid und im übrigen Helium.

8. Verwendung eines medizinischen Gasgemischs nach Anspruch 7 mit 30% Sauerstoff, 2% Kohlendioxid und 68% Helium.

## Revendications

1. Utilisation d'un mélange médical de gaz dans la préparation d'un médicament pour la visualisation et/ou le traitement de tumeurs chez des patients inhalant le mélange, le mélange comprenant de 20 à 70 % d'oxygène, de 1 à 8 % de dioxyde de carbone, le reste étant de l'hélium.

2. Utilisation d'un mélange médical de gaz selon la Revendication 1, dans lequel la teneur en oxygène est de 20 à 40 %.

3. Utilisation d'un mélange médical de gaz selon la Revendication 1 ou la Revendication 2, dans lequel la teneur en oxygène est de 25 à 35 %.

4. Utilisation d'un mélange médical de gaz selon l'une quelconque des Revendications précédentes, dans lequel la teneur en dioxyde de carbone est de 1 à 5 %.

5. Utilisation d'un mélange médical de gaz selon l'une quelconque des Revendications précédentes, dans lequel la teneur en dioxyde de carbone est de 1 à 4 %.

6. Utilisation d'un mélange médical de gaz selon l'une quelconque des Revendications précédentes, dans lequel la teneur en dioxyde de carbone est inférieure à 3 %.

7. Utilisation d'un mélange médical de gaz selon la Revendication 1, contenant de 25 à 35 % d'oxygène, de 1 à 3 % de dioxyde de carbone, le reste étant de l'hélium.

8. Utilisation d'un mélange médical de gaz selon la Revendication 7, contenant 30 % d'oxygène, 2 % de dioxyde de carbone et 68 % d'hélium.
